# EUROPÄISCHE PATENTSCHRIFT

(11) **EP 1 141 080 B1**
(45) Veröffentlichungstag und Bekanntmachung des Hinweises auf die Patenterteilung: **15.10.2003**
(21) Anmeldenummer: 99961062.9
(22) Anmeldetag: 07.12.1999
(51) Int. Cl.: C08G 64/14, A63H 9/00, A61B 19/00

(54) **VERWENDUNG VON OLIGOMEREN JODIERTEN POLYCARBONATEN**
USE OF OLIGOMERIC IODINATED POLYCARBONATES
UTILISATION DE POLYCARBONATES OLIGOMERES IODES

(30) Priorität: 18.12.1998 DE 19858774
(43) Veröffentlichungstag der Anmeldung: 10.10.2001
(73) Patentinhaber: Bayer Aktiengesellschaft, 51368 Leverkusen (DE)
(72) Erfinder: BRENNER, Axel, D-40479 Düsseldorf (DE); DÖBLER, Martin, D-40593 Düsseldorf (DE); KÖHLER, Walter, D-47239 Duisburg (DE); NEUMANN, Siegfried, D-47918 Tönisvorst (DE)
(86) Internationale Anmeldenummer: EP9909557
(87) Internationale Veröffentlichungsnummer: WO00037530

(56) Entgegenhaltungen:
- EP-A- 0 311 008
- DE-A- 1 720 812
- DE-A- 19 545 289
- DE-A- 19 726 191
- US-A- 3 382 207
- US-A- 4 182 787
- DATABASE WPI Section Ch, Week 199619 Derwent Publications Ltd., London, GB; Class A96, AN 1996-182600 XP002132277 & JP 08 057034 A (TERUMO CORP), 5. März 1996 (1996-03-05)

## Beschreibung

Gegenstand der Erfindung ist die Verwendung von Oligocarbonaten mit jodhaltigen Endgruppen für Formteile mit hohem Röntgenkontrast, besonders für Spielzeug und medizinische Teile.

Für den medizinischen Bereich und auch für Kinderspielzeug werden Materialien mit möglichst hoher Transparenz und guter Mechanik gesucht, die bei Röntgenuntersuchungen im Körper detektiert werden können. Im Gegensatz zu metallischen Gegenständen sind Gegenstände aus Kunststoff in der Regel nicht oder nur schlecht im Röntgenbild, besonders hinter Knochen, erkennbar. Durch geeignete Zusätze können solche Formmassen röntgenkontrastierbar gemacht werden.

In US 3,469,704 und DE-A 17 20 812 wurden transparente Kunststofformmassen aus Polycarbonaten mit jodhaltigen Endgruppen beschrieben.

Der Syntheseaufwand ist aber gerade bei Polymeren mit hohem Molekulargewicht u.U. recht hoch und der zu erreichende Jodgehalt im Formkörper ist teilweise gering.

Für kommerziell erhältliche jodhaltige Verbindungen könnten sich Probleme bei der Kompatibilisierung, der Farbstabilität und der Migration von Molekülen ergeben.

Für transparente Kunststoffteile, besonders Spielzeug und medizinische Teile, steht im Stand der Technik bis jetzt also noch keine adäquate röntgenkontratierbare Kunststoffmasse zur Verfügung.

Aufgrund seiner hervorragenden mechanischen Eigenschaften wurde bislang Polycarbonat insbesondere für mechanisch hochbeanspruchte und transparente Spielzeugteile verwendet. Es soll nun ein Kunststofftyp entwickelt werden, der bei unverändert hoher Transparenz und möglichst nur geringfügig verschlechterten mechanischen Eigenschaften im Rahmen einer herkömmlichen Röntgenaufnahme erkennbar ist. Die Schichtdicke bei der der Kunststoff noch erkennbar ist, sollte möglichst gering sein, höchstens jedoch 1.2 mm.

Die Aufgabe bestand darin Formmassen zu entwickeln, die bei guter Mechanik und Transparenz einen ausreichenden Kontrast bei Röntgenuntersuchungen besitzen. Auf den Zusatz von Schwermetallen sollte aus toxikologischen Gründen verzichtet werden, da Materialien für Kinderspielzeug gesucht waren.

Gegenstand der vorliegenden Anmeldung ist demnach die Verwendung von Oligocarbonaten mit jodhaltigen Endgruppen für Formteile mit hohem Röntgenkontrast, wobei die Oligocarbonate mehr als eine Dioleinheit aufweisen und ein Gewichtsmittel des Molekulargewichts Mw von kleiner 3 000 haben

Es sind Mischungen der oben aufgeführten jodhaltigen Verbindungen miteinander und mit anderen jodhaltigen Substanzen denkbar.

Besonders geeignet sind Oligocarbonate mit jodierten Phenolen, wie 2-Jodphenol, 3-Jodphenol, 4-Jodphenol, 2,3-Dijodphenol, 2,4-Dijodphenol, 2,5-Dijodphenol, 2,6-Dijodphenol, 3,4-Dijodphenol, 3,5-Dijodphenol, 2,3,4-Trijodphenol, 2,3,5-Trijodphenol, 2,3,6-Trijodphenol, 2,4,5-Trijodphenol, 2,4,6-Trijodphenol, 3,4,5-Trijodphenol, sowie deren alkylsubsituierte Verbindungen, als Endgruppe. Besonders bevorzugt ist als Endgruppe 2,4,6-Trijodphenol. Als Dioleinheit für die jodierten Oligocarbonate eignen sich alle in dieser Patentschrift für die Verwendung in Polycarbonat erwähnten Diole, vorzugsweise 2,2-Bis-(4-hydroxyphenyl)-propan.

Die Formteilc, besonders Spielzeug und medizinische Geräte, enthalten die oligomere Jodverbindung in solchen Mengen, daß der Jodgehalt zwischen 0,2 bis 19,9 Gew.-%, bevorzugt 0,3 bis 15 Gew.-%, besonders bevorzugt 0,4 bis 10 Gew.-% liegt.

In der Regel werden die Oligocarbonate mit jodhaltigen Endgruppen in transparenten Kunststoffen eingesetzt.
Als transparente Kunststoff werden bevorzugt transparenten Thermoplaste genutzt, besonders bevorzugt die Polymerisate von ethylenisch ungesättigten Monomeren und/oder Polykondensate von bifunktionellen reaktiven Verbindungen.

Besonders geeignete Kunststoffe sind Polycarbonate oder Copolycarbonate, bevorzugt nichthalogenierte Polycarbonate oder Copolycarbonate, auf Basis von Diphenolen. Die erfindungsgemäßen Oligomere können aber auch mit Poly- oder Copolyacrylaten und Poly- oder Copolymethacrylaten wie z.B. Poly- oder Copolymethylmethacrylat, aber auch als Copolymere mit Styrol wie z.B. transparentes Polystyrolacrylnitril (SAN) verwendet werden.

Ferner könne sie in transparente Cycloolefine, Poly- oder Copolykondensate der Terephthalsäure, wie z.B. Poly- oder Copolyethylenterephthalat (PET oder CoPET) oder glycol-modifiziertes PET (PETG) eingemischt werden.

Der Fachmann erzielt ausgezeichnete Resultate mit Polycarbonaten oder Copolycarbonaten.

Thermoplastische, aromatische Polycarbonate im Sinne der vorliegenden Erfindung sind sowohl Homopolycarbonate als auch Copolycarbonate; die Polycarbonate können in bekannter Weise linear oder verzweigt sein.

Die Herstellung der Oligocarbonate und Polycarbonate, bzw. Copolycarbonate erfolgt in bekannter Weise aus Diphenolen, Kohlensäurederivaten, gegebenenfalls Kettenabbrechern und gegebenenfalls Verzweigern.

Einzelheiten der Herstellung von Polycarbonaten sind in vielen Patentschriften seit etwa 40 Jahren niedergelegt. Beispielhaft sei hier nur auf Schnell, "Chemistry and Physics of Polycarbonates", Polymer Reviews, Volume 9, Interscience Publishers, New York, London, Sydney 1964, auf D. Freitag, U. Grigo, P.R. Müller, H. Nouvertne', BAYER AG, "Polycarbonates" in Encyclopedia of Polymer Science and Engineering, Volume 11, Second Edition, 1988. Seiten 648-718 und schließlich auf Dres. U. Grigo, K. Kirchner und P.R. Müller "Polycarbonate" in Becker/Braun, Kunststoff Handbuch, Band 3/1, Polycarbonate, Polyacetale, Polyester, Celluloseester, Carl Hanser Verlag München, Wien 1992, Seiten 117-299 verwiesen.

Für die Herstellung der Polycarbonate geeignete Diphenole sind beispielsweise Hydrochinon, Resorcin, Dihydroxydiphenyle, Bis-(hydroxyphenyl)-alkane, Bis(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, á,á'-Bis-(hydroxyphenyl)-diisopropylbenzole, sowie deren kernalkylierte und kernhalogenierte Verbindungen.

Bevorzugte Diphenole sind 4,4'-Dihydroxydiphenyl, 2,2-Bis-(4-hydroxyphenyl)-propan, 2,4-Bis-(4-hydroxyphenyl)-2-methylbutan, 1,1-Bis-(4-hydroxyphenyl)-p-diisopropylbenzol, 2,2-Bis-(3-methyl-4-hydroxyphenyl)-propan, 2,2-Bis-(3-chlor-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,1-Bis-(3,5-dimethyl-4-hydroxyphenyl)-p-diisopropylbenzol, 2,2-Bis-(3,5-dichlor-4-hydroxyphenyl)-propan, 2,2-Bis-(3,5-dibrom-4-hydroxyphenyl)-propan und 1,1-Bis-(4-hydroxyphenyl)-3,3,5-trimethylcyclohexan.

Besonders bevorzugte Diphenole sind 2,2-Bis-(4-hydroxyphenyl)-propan, 2,2-Bis-(3,5-dimethyl-4-hydroxyphenyl)-propan, 2,2-Bis-(3,5-dichlor-4-hydroxyphenyl)-propan, 2,2-Bis-(3,5-dibrom-4-hydroxyphenyl)-propan, 1,1-Bis-(4-hydroxyphenyl)-cyclohexan und 1,1-Bis-(4-hydroxyphenyl)-3,3,5-trimethylcyclohexan.

Diese und weitere geeignete Diphenole sind z.B. in den US-PS 3 028 635, 2 999 835, 3 148 172, 2 991 273, 3 271 367, 4 982 014 und 2 999 846, in den deutschen Offenlegungsschriften 1 570 703, 2 063 050, 2 036 052, 2 211 956 und 3 832 396, der französischen Patentschrift 1 561 518, in der Monographie "H. Schnell, Chemistry and Physics of Polycarbonates, Interscience Publishers, New York 1964" sowie in den japanischen Offenlegungsschriften 62039/1986, 62040/1986 und 105550/1986 beschrieben.

Im Falle der Homopolycarbonate ist nur ein Diphenol eingesetzt, im Falle der Copolycarbonate sind mehrere Diphenole eingesetzt.

Geeignete Kohlensäurederivate sind beispielsweise Phosgen oder Diphenylcarbonat.

Geeignete Kettenabbrecher sind sowohl Monophenole als auch Monocarbonsäuren. Geeignete Monophenole sind Phenol selbst, Alkylphenole wie Kresole, p-tert.-Butylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol und 2,4,6-Tribromphenol 2,4,6-Trijodphenol, p-Jodphenol, sowie deren Mischungen.

Bevorzugter Kettenabbrecher ist p-tert.-Butylphenol.

Geeignete Monocarbonsäuren sind Benzoesäure, Alkylbenzoesäuren und Halogenbenzoesäuren.

Bevorzugte Kettenabbrecher sind die Phenole der Formel (I) 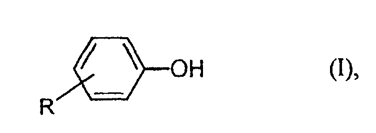 worin R Wasserstoff, tert.-Butyl oder ein verzweigter oder unverzweigter C₈- und/oder C₉-Alkylrest ist.

Die Menge an einzusetzendem Kettenabbrecher beträgt 0,1 Mol-% bis 5 Mol-%, bezogen auf Mole an jeweils eingesetzten Diphenolen. Die Zugabe der Kettenabbrecher kann vor, während oder nach der Phosgenierung erfolgen.

Geeignete Verzweiger sind die in der Polycarbonatchemie bekannten tri- oder mehr als trifunktionellen Verbindungen, insbesondere solche mit drei oder mehr als drei phenolischen OH-Gruppen.

Geeignete Verzweiger sind beispielsweise Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-[4,4-bis-(4-hydroxyphenyl)-cyclohexyl]-propan, 2,4-Bis-(4-hydroxyphenyl-isopropyl)-phenol, 2,6-Bis-(2-hydroxy-5'-methyl-benzyl)-4-methylphenol, 2-(4-Hydroxyphenyl)-2-(2,4-dihydroxyphenyl)-propan, Hexa-(4-(4-hydroxyphenyl-isopropyl)-phenyl)-orthoterephthalsäureester, Tetra-(4-hydroxyphenyl)-methan, Tetra-(4-(4-hydroxyphenyl-isopropyl)-phenoxy)-methan und 1,4-Bis-(4',4"-dihydroxytriphenyl)-methyl)-benzol sowie 2,4-Dihydroxybenzoesäure, Trimesinsäure, Cyanurchlorid und 3,3-Bis-(3-methyl-4-hydroxyphenyl)-2-oxo-2,3-dihydroindol.

Die Menge der gegebenenfalls einzusetzenden Verzweiger beträgt 0,05 Mol-% bis 2 Mol-%, bezogen wiederum auf Mole an jeweils eingesetzten Diphenolen.

Die Verzweiger können entweder mit den Diphenolen und den Kettenabbrechern in der wäßrig alkalischen Phase vorgelegt werden, oder in einem organischen Lösungsmittel gelöst vor der Phosgenierung zugegeben werden. Im Falle des Umesterungsverfahrens werden die Verzweiger zusammen mit den Diphenolen eingesetzt.

Alle diese Maßnahmen zur Herstellung der thermoplastischen Polycarbonate sind dem Fachmann geläufig.

Der Kunststoff macht naturgemäß die Hauptmenge der Zusammensetzungen aus, so daß er in der Regel in Mengen zwischen 80,0 und 99,9 Gew.-%, bevorzugt 85,0 und und 99,9 Gew.-%, besonders bevorzugt 90 und 99,9 aber auch 85,0 und 98 Gew.-%, ganz besonders bevorzugt zwischen 90 und 97 aber auch zwischen 93 und 98 Gew.-% auf das Formteil vorhanden ist.

In einer bevorzugten Ausführungsform ist der Jodgehalt im Formteil zwischen 0,2 bis 19,9 Gew.-%, bevorzugt 0,3 bis 15 Gew.-%, besonders bevorzugt 0,4 bis 10 Gew.-%.
Es kann aber auch wünschenswert sein, wenn der Jodgehalt im Formteil zwischen 20,1 bis 30 Gew.-% beträgt.

Es ist zur Erreichung von verbesserten Zusammensetzungen möglich, daß zusätzlich noch mindestens ein weiterer in thermoplastischen Kunststoffen, bevorzugt Polyund Copolycarbonaten, üblicherweise vorhandener Zusatzstoff wie z.B. Stabilisatoren (wie z.B. in EP 0 839 623 A1 oder EP 0 500 496 A1 beschrieben) besonders Thermostabilisatoren, insbesondere organische Phosphite oder Phosphine, z.B. Triphenylphosphin, Entformungsmittel, beispielsweise Fettsäureester des Glycerins oder Tetramethanolmethans, wobei ungesättigte Fettsäure auch ganz oder teilweise epoxidiert sein können, insbesondere Glycerinmonostearat oder Pentaerytrittetrastearat (PETS), Flammschutzmittel, Antistatika, UV-Absorber, beispielsweise Triazole, Füllmittel, Schaummittel, Farbstoffen, Pigmente, optische Aufheller, Umesterungskatalysatoren und Nukleierungsmittel o.ä., bevorzugt in Mengen von jeweils bis zu 5 Gew.-%, bevorzugt 0,01 bis 5 Gew.-% bezogen auf die gesamte Mischung, besonders bevorzugt 0,01 Gew.-% bis 1 Gew.-% bezogen auf die Menge Kunststoff enthalten ist.

Auch Mischungen dieser Zusatzstoffe sind möglich.

Besonders gute Eigenschaften erzielt man mit UV-Stabilisatoren der Triazolreihe. Hier sind besonders zu nennen:2-(3',5-'Bis-(1,1-dimethylbenzyl)-2'-hydroxyphenyl)-benzotriazol, 2-(2'-Hydroxy-5'-(*tert*-octyl)-phenyl)-benzotriazol, 2-(2'-Hydroxy-3'-(2-butyl)-5'-(*tert*-butyl)-phenyl)-benzotriazol, Bis-(3-(2H-benztriazolyl)-2-hydroxy-5-*tert*-octyl)methan, 2-(4-Hexoxy-2-hydroxyphenyl)-4,6-diphenyl-1,3,5-triazin, sowie Benzophenone, wie z.B. 2,4-Dihydroxy-benzophenon.

Die so erhaltenen röntgenopaken Polymerzusammensetzungen können nach den üblichen Methoden, wie z.B. Heißpressen, Spinnen, Extrudieren oder Spritzgießen, in geformte Gegenstände überführt werden, wie z.B. Spielzeugteile, aber auch Fasern, Folien, Bändchen, Platten, Stegplatten, Gefäße, Rohre und sonstige Profile. Die Polymerzusammensetzungen können auch zu Gießfolien verarbeitet werden. Die Erfindung betrifft daher weiterhin die Verwendung der erfindungsgemäßen Polymerzusammensetzungen zur Herstellung eines geformten Gegenstandes. Von Interesse ist auch die Verwendung von Mehrschichtsystemen. Hierbei wird die erfindungsgemäße Polymerenzusammensetzung mit einem relativ hohen Gehalt an jodhaltigen Zusätzen in dünner Schicht auf einen geformten Gegenstand aus einem Polymer, welches röntgentransparent ist, aufgebracht. Das Aufbringen kann zugleich oder unmittelbar danach mit der Formgebung des Grundkörpers geschehen, z.B. durch Coextrusion oder Mehrkomponentenspritzguß. Das Aufbringen kann aber auch auf den fertig geformten Grundkörper geschehen, z.B. durch Lamination mit einem Film oder durch Beschichtung mit einer Lösung. Die Formmassen sind besonders für transparente Kinderspielzeugteile oder für medizinische Anwendungen, wie Medizinische Geräte, beispielsweise Sonden oder Gelenkteile geeignet. Ganz besonders geeignet sind solche Formteile hierbei für kleine Teile von Kinderspielzeug.

### Beispiele

### Herstellung der erfindungsgemäßen jodierten Oligocarbonate

161.1 g (0,11 mol) Oligo-[2,2-Bis-(4-hydroxyphenyl)-propancarbonat]chlorkohlensäureester ( Polymerisationsgrad ca. 5) werden in 1000 g Dichlormethan gelöst. Bei 20-25°C wird unter Rühren eine klare Lösung bestehend aus 89.1g (0,189 mol) 2,4,6-Triiodphenol, 35.6 g (0.40 mol) 45 %ige Natronlauge und 800 g Wasser zugegeben. Nach 5 Minuten werden 0.71 g N-Ethylpiperidin hinzugefügt und 30 Minuten intensiv gerührt. Die Dichlormethan-Phase wird von der wässrigen getrennt und elektrolytfrei gewaschen. Das Lösungsmittel wird abgedampft und die eingeengte Lösung bei 120°C im Wasserstrahlvakuum getrocknet.
Ausbeute: 202 g farbloser Feststoff
Analysen: phenolisches OH: 160 mg/kg, verseifbares Cl: <0.2 mg/kg
Jodgehalt: ca. 27 %

### Beispiel 1:

94.7 Teile Polycarbonat (Makrolon2808®, Bayer AG) werden zusammen mit 5,9 Gew.-% erfindungsgemäßen Oligocarbonaten (Trijodphenol-Endgruppen) und 0,5 Gew.-% Entformungsmittel PETS bei 280°C mit einem Zweischneckenextruder kompoundiert und anschließend zu Prüfstäben mit unterschiedlicher Dicke verspritzt. Die Eigenschaften dieser Formkörper sind in Tabelle 1 zusammengefaßt:

### Tabelle 1:

E-Modul: 2500 N/mm²
Reißdehnung: 119 %
Tg = 143°C
Iodgehalt der Formkörper: 1.6 %
Prüfstab 1.2 mm Dicke: röntgendetektierbar
Prüfstab 1.6 mm Dicke: röntgendetektierbar
Prüfstab 2.4 mm Dicke: röntgendetektierbar
Prüfstab 3.2 mm Dicke: röntgendetektierbar
Die Röntgendetektierbarkeit wurde mit den im medizinischen Bereich üblichen Parametern gezeigt.

Die Transparenz dieser Formkörper betrug jeweils mehr als 85 %. Selbst mit 85 Gew.-% Polycarbonat, 14, 5 Gew.-% erfindungsgemäßem Oligomer (Trijodphenol-Endgruppen) und 0,5 Gew.-% Entformungsmittel PETS sind die Prüfkörper noch vollkommen transparent.

Diese Formkörper können somit bei einer herkömmlichen medizinischen Röntgenuntersuchung im menschlichen Körper selbst im Schatten von Knochen detektiert werden.

Der Zusatz von niedermolekularen Jodverbindungen bewirkt eine Weichmachung des Materials. Dies ist am verringerten Glaspunkt und an der niedrigeren Lösungsviskosität erkennbar. Die Glasübergangstemperatur Tg von Beispiel 1 bleibt jedoch praktisch unverändert hoch, so daß eine hohe Wärmeformbeständigkeit erhalten bleibt.

## Patentansprüche

1. Verwendung von Oligocarbonaten mit jodhaltigen Endgruppen für Formteile mit hohem Röntgenkontrast, **dadurch gekennzeichnet, daß** die Oligocarbonate mehr als eine Dioleinheit aufweisen und ein Gewichtsmittel des Molekulargewichts Mw von kleiner 3 000 haben.

2. Verwendung nach Anspruch 1, **dadurch gekennzeichnet, daß** in den Formteilen zusätzlich mindestens ein transparenter Thermoplast in Mengen von 80 bis 99,9 Gew.-%, bezogen auf das Formteil, enthalten ist.

3. Verwendung nach Anspruch 2 **dadurch gekennzeichnet, daß** der transparente Thermoplast in Mengen von 85 bis 98 Gew.-%, bezogen auf das Formteil, enthalten ist.

4. Verwendung nach Anspruch 2 **dadurch gekennzeichnet, daß** der transparente Thermoplast in Mengen von 90 bis 97 Gew.-%, bezogen auf das Fonnteil, enthalten ist.

5. Verwendung nach Anspruch 2 **dadurch gekennzeichnet, daß** als transparenter Thermoplast ein nichthalogeniertes Polycarbonat oder Copolycarbonat verwendet wird.

6. Verwendung nach mindestens einem der vorgenannten Ansprüche, **dadurch gekennzeichnet, daß** der Jodgehalt im Formteil zwischen 0,2 bis 19,9 Gew.-% beträgt.

7. Verwendung nach mindestens einem der vorgenannten Ansprüche, **dadurch gekennzeichnet, daß** der Jodgehalt im Formteil zwischen 0,3 bis 15 Gew.-% beträgt.

8. Verwendung nach mindestens einem der vorgenannten Ansprüche, **dadurch gekennzeichnet, daß** der Jodgehalt im Formteil zwischen 20,1 bis 30 Gew.-% beträgt

9. Spielzeug, enthaltend Oligocarbonate mit jodhaltigen Endgruppen, **dadurch gekennzeichnet, daß** die Oligocarbonate mehr als eine Dioleinheit aufweisen und ein Gewichtsmittel des Molekulargewichts Mw von kleiner 3 000 haben, zur Verbesserung des Röntgenkontrastes.

10. Medizinische Geräte wie Sonden oder Gelenkteile enthaltend Oligocarbonate mit jodhaltigen Endgruppen, **dadurch gekennzeichnet, daß** die Oligocarbonate mehr als eine Dioleinheit aufweisen und ein Gewichtsmittel des Molekulargewichts Mw von kleiner 3 000 haben, zur Verbesserung des Röntgenkontrastes.

## Claims

1. Use of oligocarbonates with iodine-containing terminal groups for moulded items with a high X-ray contrast, **characterised in that** the oligocarbonates contain more than one diol unit and have a weight average molecular weight Mw of less than 3,000.

2. Use according to Claim 1, **characterised in that** at least one transparent thermoplastic material is also present in the moulded items, in amounts of 80 to 99.9 wt.% with respect to the moulded item.

3. Use according to Claim 2, **characterised in that** the transparent thermoplastic material is present in amounts of 85 to 98 wt.%, with respect to the moulded item.

4. Use according to Claim 2, **characterised in that** the transparent thermoplastic material is present in amounts of 90 to 97 wt.%, with respect to the moulded item.

5. Use according to Claim 2, **characterised in that** a non-halogenated polycarbonate or copolycarbonate is used as the transparent thermoplastic material.

6. Use according to at least one of the previously mentioned Claims, **characterised in that** the iodine content in the moulded item is between 0.2 and 19.9 wt.%.

7. Use according to at least one of the previously mentioned Claims, **characterised in that** the iodine content in the moulded item is between 0.3 and 15 wt.%.

8. Use according to at least one of the previously mentioned Claims, **characterised in that** the iodine content in the moulded item is between 20.1 and 30 wt.%.

9. Toys containing oligocarbonates with iodine-containing terminal groups, **characterised in that** the oligocarbonates contain more than one diol unit and have a weight average molecular weight Mw of less than 3,000 to improve the X-ray contrast.

10. Medical equipment such as probes or joint parts containing oligocarbonates with iodine-containing terminal groups, **characterised in that** the oligocarbonates contain more than one diol unit and have a weight average molecular weight Mw of less than 3,000 to improve the X-ray contrast.

## Revendications

1. Utilisation d'oligocarbonates munis de groupes terminaux contenant de l'iode pour des pièces moulées avec un contraste radiographique élevé, **caractérisée en ce que** les oligocarbonates présentent plus d'une unité diol et ont une moyenne massique du poids moléculaire Mw inférieure à 3000.

2. Utilisation selon la revendication 1, **caractérisée en ce qu'**est contenu en plus dans les pièces moulées au moins un thermoplaste transparent dans des quantités de 80 à 99,9 % en poids, rapportés à la pièce moulée.

3. Utilisation selon la revendication 2, **caractérisée en ce que** le thermoplaste transparent est contenu dans des quantités de 85 à 98 % en poids, rapportés à la pièce moulée.

4. Utilisation selon la revendication 2, **caractérisée en ce que** le thermoplaste transparent est contenu dans des quantités de 90 à 97 % en poids, rapportés à la pièce moulée.

5. Utilisation selon la revendication 2, **caractérisée en ce que** l'on utilise comme thermoplaste transparent un polycarbonate ou un copolycarbonate non halogéné.

6. Utilisation selon l'une quelconque des revendications précédentes, **caractérisée en ce que** la teneur en iode dans la pièce moulée est comprise entre 0,2 et 19,9 % en poids.

7. Utilisation selon au moins l'une quelconque des revendications citées précédemment, **caractérisée en ce que** la teneur en iode dans la pièce moulée est comprise entre 0,3 et 15 % en poids.

8. Utilisation selon au moins l'une quelconque des revendications citées précédemment, **caractérisée en ce que** la teneur en iode dans la pièce moulée est comprise entre 20,1 et 30 % en poids.

9. Jouet contenant des oligocarbonates munis de groupes terminaux contenant de l'iode, **caractérisé en ce que** les oligocarbonates présentent plus d'une unité diol et ont une moyenne massique du poids moléculaire Mw inférieure à 3000 pour améliorer le contraste radiographique.

10. Appareils médicaux comme des sondes ou des pièces d'articulation contenant des oligocarbonates munis de groupes terminaux contenant de l'iode, **caractérisés en ce que** les oligocarbonates présentent plus d'une unité diol et ont une moyenne massique du poids moléculaire Mw inférieure à 3000 pour améliorer le contraste radiographique.
